# EUROPEAN PATENT SPECIFICATION

(11) **EP 2 663 220 B1**
(45) Date of publication and mention of the grant of the patent: **17.07.2019**
(21) Application number: 12701030.4
(22) Date of filing: 10.01.2012
(51) Int. Cl.: A61B 1/267, A61B 6/03, A61B 90/00, G06T 19/00

(54) **VIRTUAL ENDOSCOPIC IMAGING WITH HIGH RISK STRUCTURE HIGHLIGHTING**
VIRTUELLE ENDOSKOPISCHE BILDGEBUNG MIT HERVORHEBUNG VON HOCHRISIKOSTRUKTUREN
IMAGERIE ENDOSCOPIQUE VIRTUELLE À MISE EN ÉVIDENCE DE STRUCTURE À HAUT RISQUE

(30) Priority: 14.01.2011 US 201161432763 P
(43) Date of publication of application: 20.11.2013
(73) Proprietor: Koninklijke Philips N.V., 5656 AE Eindhoven (NL); Philips Intellectual Property & Standards GmbH, 20099 Hamburg (DE)
(72) Inventor: KLINDER, Tobias, 5656 AE Eindhoven (NL); WIEMKER, Rafael, 5656 AE Eindhoven (NL); XU, Sheng, 5656 AE Eindhoven (NL)
(74) Representative: Steffen, Thomas
(86) International application number: PCT/IB2012/050116
(87) International publication number: WO 2012/095788

(56) References cited:
- WO-A1-2008/095068
- WO-A2-2005/008591
- DE-A1-102006 003 179
- US-A1- 2004 015 070
- US-A1- 2009 156 895
- US-B1- 6 346 940
- C.RIEDER,M.SCHWIER,A.WEIHUSEN,S.ZIDOWITZ,H -O.PEITGEN: "Visualization of Risk Structures for interactive planning of image guided radiofrequency ablation of liver tumors", SPIE, PO BOX 10 BELLINGHAM WA 98227-0010 USA, vol. 7261, 2009, pages 726134-1-726134-9, XP040495088,

## Description

The invention relates to the field of medical imaging and more particularly to a method and system for highlighting high risk structures in a virtual endoscopic image.

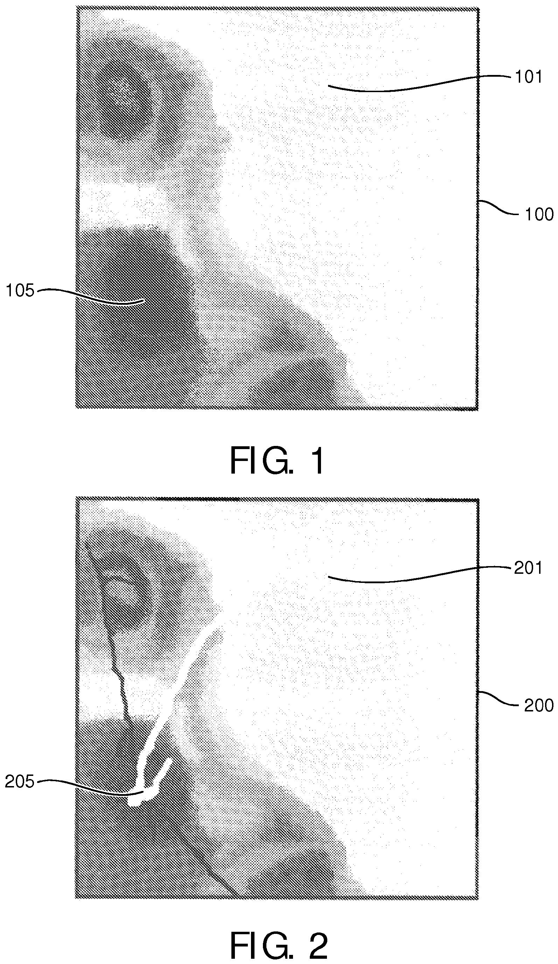

Bronchoscopies with or without transbronchial biopsies are a common interventional procedure, in which a bronchoscope camera is advanced into the tracheobronchial airway tree for diagnostic and optionally therapeutic purposes. The bronchoscope provides an image 100 from inside of the bronchial, as shown in Fig. 1. However, bronchoscopy typically suffers from the problem that navigation within the bronchial tree is difficult for the physician during an intervention. The physician can easily get lost and be unable to navigate to the position that he/she was originally interested in. In order to assist the planning, as well as the real-time guidance of such an intervention, a prior thoracic Computed Tomography (CT) scan of the patient may be used to compute virtual endoluminal renderings 200 which closely resemble the optical camera images from the real bronchoscope, as shown in Fig. 2. The virtual rendering can be registered to the current bronchoscopic image. The CT scan, as well as the virtual endoluminal renderings derived from it can be used to generate a virtual fly-through of the intervention and to plan the path to a certain anomaly, tumor, lymph node, airway constriction, or the like.

The virtual fly-through generates images as if the physician was inside the bronchial, thus showing only the bronchial tree, and more particularly, the bronchial wall from inside the bronchial. However, the physician is interested in structures that lie behind the bronchial wall, especially high risk structures such as large blood vessels. This information is particularly important when the physician plans to cut through the bronchial wall, such as for tumor biopsies, or to target a lymph node. In this case, the physician must make sure that no other (or at least no important) structures are hit when he/she cuts through the bronchial wall.

Currently, a physician must rely upon his/her expert knowledge about the location of important nearby structures when cutting through the bronchial wall. However, this only leads to a rough estimate of the locations of important nearby structures. It can not provide an exact location and can not account for variations among patients, and therefore, it cannot be considered to be very secure.

Alternatively, a physician may navigate to an estimated position in bronchial, then change the transparency setting for the bronchial wall. One problem with this approach is that is that physician cannot navigate with a low transparency, as then the visualization gets quite confusing. Thus, physician has to repeatedly stop, change transparency and look if there is a structure. Moreover, structures are not easy to see in a transparency view. Nor is easy to determine the distance to a structure in a transparency view, or if it will be hit perform a biopsy at that point.

Document US2009/0156895 discloses a method and a system for virtual bronchoscopy, based on multidetector computed tomography (MDCT) acquisition. The method and the system assist a user to sample a ROI, by visualizing obstacles, which are located beyond the airway walls. If the obstacles are within a pre-determined safety envelope, they are depicted in a unique color, informing the physician of poor locations for sampling the ROI.

The present invention provides a method, a system, and a program product for highlighting high risk structures in a virtual rendering for use during an intervention procedure in a body lumen, according to claims 1,10 and 13, respectively. By highlighting areas of a body lumen wall that are too close to a high risk structure in the virtual rendering, the physician performing an intervention can visualize areas where it is not safe to cut through the lumen wall. The highlighted virtual rendering may be presented during fly through before performing an intervention or during an intervention.

According to one embodiment of the present invention a method is provided for highlighting high risk structures in a virtual rendering for use during an intervention al procedure in a body lumen. The physician performs a pre-procedural CT scan of the body lumen. Using the CT scan, a modeling program segments the body lumen and segments a high risk structure external to the body lumen. The modeling program determines relative distances between the body lumen wall and the high risk structure and compares the distances to a marking criteria. The modeling program creates a virtual rendering of the inside of the body lumen corresponding to an interventional camera image; and marks areas of a wall of the virtual rendering of the body lumen corresponding to the comparison of the relative distance to the marking criteria.

According to one embodiment areas of the body lumen wall are marked that are within a predefined distance of the high risk structure. Areas of the body lumen wall may be marked by painting voxels within a predefined distance to the high risk structure a predefined color.

According to one embodiment a plurality of colors may be used to mark the lumen walls. In one embodiment the plurality of colors may be used to differentiate between multiple distance thresholds. Areas of the body lumen wall are painted one of a plurality of colors corresponding to the distance of the high risk structure to each respective area being within one of a plurality of predefined limits. According to another embodiment a plurality of colors may be used to differentiate which of a plurality of structures is proximate an area of the body lumen wall. An area of the body lumen wall may be painted a color corresponding to one of a plurality of high risk structures within a predefined distance to the area of the body lumen wall.

According to one embodiment the body lumen wall in the virtual rendering comprises a plurality of voxels. Each voxel of the body lumen wall is painted if the distance between the voxel and a high risk structure is within a pre-defined threshold value. In one embodiment the voxels are painted with a translucent overlay, allowing the physician to visualize the underlying body lumen wall.

According to one embodiment points on the body lumen wall are marked if distance between the point on the body lumen wall and the high risk structure, measured at an angle at which a physician plans to cut, is within a pre-defined threshold value.

According to one embodiment the body lumen is a traeceobroncial airway tree. In alternative embodiments, the body lumen may be another tubular organ, such as a colon.

According to one embodiment the high risk structure is blood vessels. According to alternative embodiments other high risk organs may be identified. Also, areas of the body lumen wall within a threshold distance of target structures such as tumors or lymph nodes may be marked.

According to one embodiment of the present invention a system is provided for highlighting high risk structures in a virtual rendering for use during an intervention procedure in a body lumen. The system comprises: a processor; a memory operably connected with the processor; and a display operably connected with the processor; wherein the memory has encoded thereon a modeling program, which is executed by the processor to receive pre-procedural CT-scans, segment a body lumen and a high risk structure outside the body lumen from the pre-procedural CT scans, generate a virtual rendering of an endoscopic view of the body lumen from, determine relative distances between the body lumen wall and the high risk structure, mark voxels on a wall of the body lumen in the virtual rendering that are within a threshold distance from the high risk structure, and display the virtual rendering with marking to highlight high risk structures on the display. The system may be realized as an imaging work station.

According to one embodiment the system further comprising an endoscope operably connected to the processor, whereby the virtual rendering can be registered to a live endoscopic image generated by the endoscope and presented along side of the endoscopic image.

According to one embodiment of the present invention a computer program product is provided comprising a non-transitory, computer-readable storage media having encoded thereon: program instructions for performing a CT scan of the body lumen; program instructions for segmenting the body lumen; program instructions for segmenting a high risk structure; program instructions for determining relative distances between the body lumen wall and the high risk structure located outside of the body lumen; program instructions for comparing the distances to a marking criteria; program instructions for creating a virtual rendering of the inside of the body lumen corresponding to an interventional camera image; and program instructions for marking areas of the body lumen wall in the virtual rendering corresponding to the comparison of the relative distance to the marking criteria.

The features and advantages of the invention will be more clearly understood from the following detailed description of the preferred embodiments when read in connection with the accompanying drawing. Included in the drawing are the following figures:
Fig. 1 is an image from a bronchoscope according to the prior art;
Fig 2 is a virtual rendering of a traecheobronchial airway according to the prior art;
Fig. 3 a block diagram of a system for marking areas of a bronchial wall of a virtual rendering to indicate proximity to a high risk structure according to an embodiment of the present invention;
Fig. 4 is a brachial tree image generated from a CT scan with an image of blood vessels registered to the bronchial tree according to various embodiments of the present invention;
Fig. 5 is a virtual image of a bronchial marked to show areas of the bronchial wall that are close to high risk structures according to an embodiment of the present invention;
Fig. 6 is a flow diagram of a method for marking areas of a bronchial wall of a virtual rendering to indicate proximity to a high risk structure according to an embodiment of the present invention; and
Fig. 7 is a flow diagram of a method for determining relative distances between the body lumen and the high risk structure according to an embodiment of the present invention.

The present invention provides a method, system, and program product for marking areas of a body lumen wall of a virtual rendering to indicate proximity to a high risk structure.

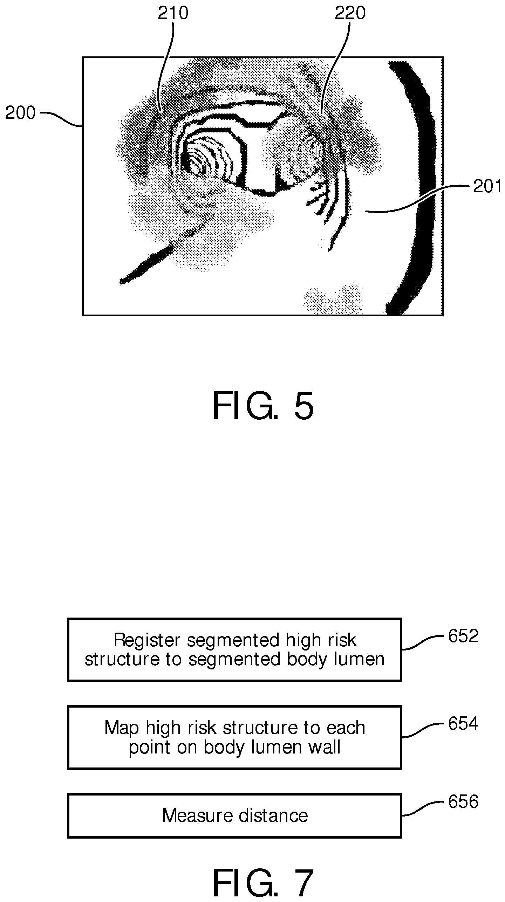

According to one embodiment of the present invention, areas of body lumen wall 201 in a virtual rendering 200 are marked to indicate proximity to a high risk structure, as shown in Fig. 5. Marked areas may areas which are very close to a high risk structure 210 making it too risky to cut in these areas. Areas 220 where it is safe to cut may also be marked. The areas 210, 220 may be marked, for example, by painting the lumen wall 201 in the appropriate area. For example, these areas may be painted with a translucent overlay that is color coded to indicate the type of area.

In the present invention, relevant information about nearby structures can be readily obtained directly from the virtual rendering 200 by observing markings 210, 220 painted on the virtual rendering 200. The markings may be provided as an optional overlay in a fly through application without significant user interaction, as little as one mouse click can provide the relevant proximity information. Thus, the physician immediately has information on the proximity of nearby high risk structures and can easily determine whether or not he/she should cut the bronchial wall 101 at a particular position.

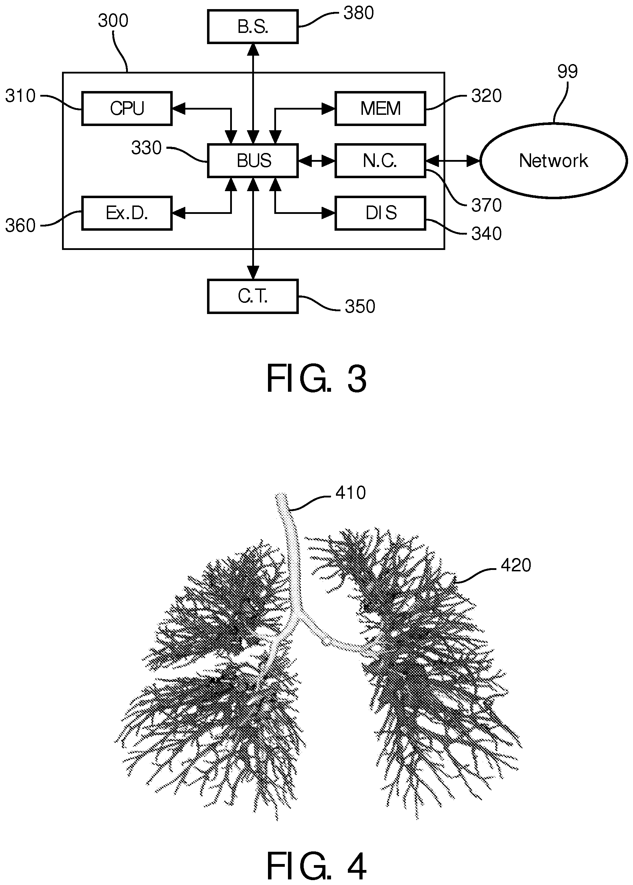

Referring now to Fig. 3, a system 300 is shown for marking areas of a bronchial wall in a virtual rendering that are proximate high risk structures. According to one embodiment, the system 300 may be realized in an imaging workstation, such as an Extended Brilliance Workspace (EBW) from Philips Electronics, N. V., Eindhoven, Netherlands. The EBW provides a graphical user interface which combines nuclear imaging such as Single Photon Emission Computer Tomography (SPECT) with Computer Tomography (CT).

According to one embodiment, the system 300 comprises a general purpose computer or a custom computing device. The system 300 comprises a central processing unit 310 that is operably connected with a memory 320 through a system bus 330 or the like. The processing unit 310 may be may be any device capable of executing program instructions, such as one or more microprocessors. The memory may be any volatile or non-volatile memory device, such as a removable disc, a hard drive, a CD, a Random Access Memory (RAM), a Read Only Memory (ROM), or the like, or any combination thereof.

A display 340 is also operably connected to the processor 310. The display may be any monitor, screen, or the like suitable for presenting a graphical user interface (GUI) capable of presenting medical images.

A modeling program 322 is encoded on the memory 320. The modeling program generates a three dimensional model of anatomical features from image data such as CT scans. The modeling program 322 may also create a rendering of the anatomical features from various perspectives, such as a rendering that closely resembles a view of a tracheobronchial airway tree from within the airway like the view from a bronchoscope camera, for example. The modeling program can also render images of other body lumens from within the lumens.

The modeling program 322 can identify high risk structures 420 outside the body lumen wall 201. Then, the modeling program can determine the distance from a point on the body lumen wall 201 to the high risk structure 420. This distance is compared to one or more threshold values. The threshold values may be a distance that enables a physician to safely cut through the body lumen wall. Then, based on the comparison of the distance to the threshold, the modeling program paints the point on the body lumen wall a color corresponding to the risk threshold that the distance falls within. The distance between the point on the body lumen wall and the high risk structure may be measured at an angle at which a physician is likely to cut. The distance determination may be performed for each point or voxel on the body lumen wall 201.

According to one embodiment of the present invention, pre-procedural CT scans 324 are encoded on memory 120 to be used for creating the three-dimensional anatomical model. The CT scans 134 may be uploaded from a storage device, such as a CD-ROM, or the like inserted in an external drive 360. Alternatively, the CT scans 324 may be received by a network connection 370 through a network 99, such as the internet or an intranet, or the like. To accomplish this, the external drive 360 or the network connection 370 may be operably connected to the memory 320 through a system bus 330 or the like. Alternatively, a CT system may be connected directly to the memory 320 through the system bus 330.

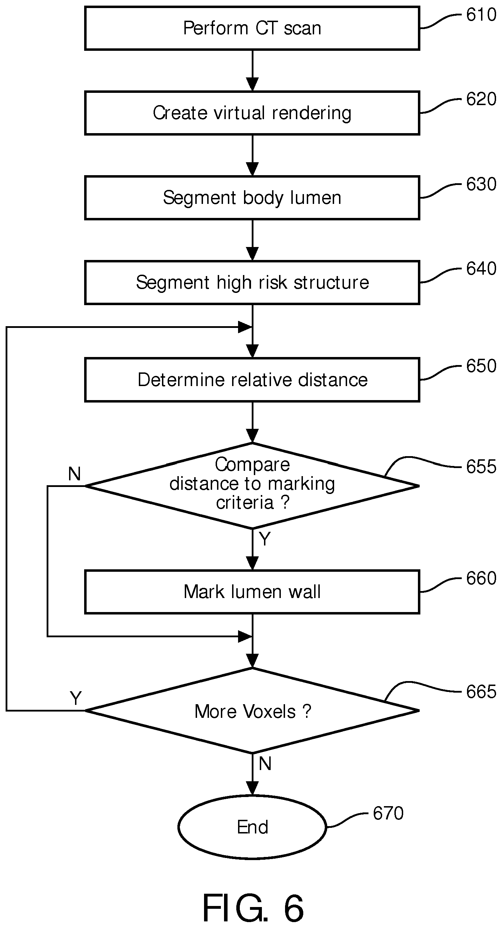

Referring now to Fig. 6, a method is shown for marking areas of a bronchial wall in a virtual rendering that are proximate high risk structures. A pre-procedure CT scan 324 is performed (Step 610). The scan 324 is then loaded into an imaging system, such as the Philips EBW. The CT scanner may be integral with the imaging system, and provide the scan directly. Alternatively, the scan may be sent to the imaging system through a network, or the scan may be recorded on a recording media, such as a CD-ROM, flash drive, or the like, which can be inserted in an appropriate drive or connector on the imaging system for access by the processor 310.

From the CT scan 324, the imaging system 300 creates a virtual rendering 200 of a body lumen 105 such as a tracheobronchial airway tree, using the modeling program 322 (Step 620). As previously described, the virtual rendering may be created by the processor 310 executing the modeling program 322 encoded on memory 320. The virtual rendering is displayed on the display 340.

The modeling program 322 segments the body lumen 205 (Step 630) and segments the high risk structure (Step 640). As shown in Fig. 4, an image of the segmented body lumen 410 (in the illustrated example - a bronchial tree) and the high risk structure 420 (in the illustrated example - blood vessels) are registered to each other separate from other neighboring structure. The segmented body lumen 410 and the segmented high risk structure 420 can be generated using methods known in the art of medical imaging.

The modeling program 322 determines the distance between a point or voxel on the body lumen wall 201 and the high risk structure (Step 650) by measuring the distance between the point on the segmented body lumen 410 and the segmented high risk structure 420 in a virtual image. The modeling program measures distances to the high risk structure for each point on the body lumen wall. Measuring distances in virtual images is known in the art and will not be discussed further herein. It should be understood that in some embodiments the distance may be measured at a predetermined angle - the angle corresponding to a cutting angle planned by the physician.

The system 300 compares the measured distance between the body lumen wall 201 and the high risk structure to a marking criteria (step 655). The marking criteria may be one or more threshold values for distances at which it is safe to cut through the body lumen wall 201. For example, the threshold may be set at 5 millimeters in one intervention procedure. If the measured distance from a point or voxel on the body lumen wall to the high risk structure is less than the threshold, for example, the system 300 marks that point or voxel by painting an overlay on it (Step 660). Multiple thresholds may be used to indicate varying degrees of risk. The overlay may be color coded to indicate the threshold that applies at that point. Alternatively, the overlay may be color coded to indicate one of a plurality of high risk structures or even an intended target, such as a tumor or lymph node of interest. Moreover, the overlay may be translucent to allow the physician to visualize the underlying lumen wall.

The threshold for marking the lumen wall 201 is application specific, and would depend on such factors as the size of an instrument used in an intervention procedure. In one example, the threshold is set at 5 millimeters measured at a cutting angle. Accordingly, the modeling program determines the distance from each voxel on the lumen wall to the high risk structure 420 measured at the cutting angle. If the distance is greater than 5 millimeters at the cutting angle for a first voxel, then that voxel is not painted. If the distance measured from a second voxel at the cutting angle is less than 5 millimeters, then that voxel is painted, making it easy for a physician performing an intervention procedure to visualize where the high risk structure is closer than 5 millimeters from the lumen wall measured at a cutting angle.

If the measured distance for the current voxel does not meet the threshold criteria, then the current voxel is not marked, and the system proceeds to step 665 to determine if there are more voxels on the body lumen wall 201.

After the measured distance at one point is compared, the system 300 determines whether or not there are additional points on the lumen wall 201 (Step 665). If there are additional voxels on the body lumen wall 201, then the system repeats the determination of relative distance (Step 650), comparison of the measured distance to the marking criteria (step 655), and marking of the voxel (Step 660), if required, for the next voxel. These steps are repeated until all voxels have been measured, compared, and marked.

If there are no additional points, then the process ends (step 670). The virtual rendering with markings to show proximity of high risk structures is shown on the display 340. This image may be used for a fly through of the procedure, and may be shown beside an actual endoscopic image during an intervention procedure.

Fig. 7 is a flow diagram of a method for determining relative distances between the body lumen and the high risk structure according to an embodiment of the present invention. The modeling program 322 registers the segmented high risk structure 420 to the segmented body lumen 410 (Step 652). As is known in the art, each segmented image will have registration marks, which can be aligned to register the images to each other.

Once the images are registered, the modeling program maps the high risk structure 420 to each point or voxel on the body lumen wall 201 (Step 654), and measures the distance from the point or voxel to the high risk structure (Step 656).

The invention can take the form of an entirely hardware embodiment, an entirely software embodiment or an embodiment containing both hardware and software elements. In an exemplary embodiment, the invention is implemented in software, which includes but is not limited to firmware, resident software, microcode, etc.

Furthermore, the invention may take the form of a computer program product accessible from a computer-usable or computer-readable medium providing program code for use by or in connection with a computer or any instruction execution system or device. For the purposes of this description, a computer-usable or computer readable medium may be any non-transient apparatus that can contain or store the program for use by or in connection with the instruction execution system, apparatus, or device.

The foregoing method may be realized by a program product comprising a machine-readable medium having a machine-executable program of instructions, which when executed by a machine, such as a computer, performs the steps of the method. This program product may be stored on any of a variety of known machine-readable medium, including but not limited to compact discs, floppy discs, USB memory devices, and the like.

The medium can be an electronic, magnetic, optical, electromagnetic, infrared, or semiconductor system (or apparatus or device). Examples of a computer-readable medium include a semiconductor or solid state memory, magnetic tape, a removable computer diskette, a random access memory (RAM), a read-only memory (ROM), a rigid magnetic disk an optical disk. Current examples of optical disks include compact disk-read only memory (CD-ROM), compact disk-read/write (CD-R/W) and DVD.

The preceding description and accompanying drawing are intended to be illustrative and not limiting of the invention. The scope of the invention is intended to encompass equivalent variations and configurations to the full extent of the following claims.

## Claims

1. A computer implemented method for highlighting high risk structures in a virtual rendering for use during an intervention procedure in a body lumen, comprising:
performing a CT scan (610) of the body lumen;
segmenting the body lumen (630);
segmenting a high risk structure, which is located outside the body lumen wall (640);
determining relative distances between the points on the body lumen wall and the high risk structure (650);
comparing the distances to a marking criteria (655);
creating a virtual rendering of the inside of the body lumen corresponding to an interventional camera image; and
marking the points of the body lumen wall of the virtual rendering of the body lumen corresponding to the comparison of the relative distance to the marking criteria (660).

2. The method of claim 1, wherein areas of the body lumen wall are marked that are within a predefined distance of the high risk structure.

3. The method of claim 2, wherein areas of the body lumen wall are marked by painting voxels within a predefined distance to the high risk structure a predefined color.

4. The method of claim 3, wherein areas of the body lumen wall are painted one of a plurality of colors corresponding to the distance of the high risk structure to each respective area being within one of a plurality of predefined limits.

5. The method of claim 3, wherein an area of the body lumen wall is painted a color corresponding to one of a plurality of high risk structures within a predefined distance to the area of the body lumen wall.

6. The method of claim 1, wherein each voxel of the body lumen wall is painted with a translucent overlay if the distance between the voxel and a high risk structure is within a pre-defined threshold value.

7. The method of claim 1, wherein points on the body lumen wall are marked if distance between the point on the body lumen wall and the high risk structure, measured at an angle at which a physician plans to cut, is within a pre-defined threshold value.

8. The method of claim 1, wherein the body lumen is a traeceobroncial airway tree.

9. The method of claim 8 wherein the high risk structure is blood vessels.

10. A system for highlighting high risk structures in a virtual rendering for use during an intervention procedure in a body lumen, comprising:
a processor (310);
a memory (320) operably connected with the processor; and
a display (340) operably connected with the processor;
wherein the memory has encoded thereon a modeling program, which is executed by the processor to receive pre-procedural CT-scans, segment a body lumen and a high risk structure, which is located outside the body lumen wall from the pre-procedural CT scans, generate a virtual rendering of an endoscopic view of the body lumen, determine relative distances between the points of the body lumen wall and the high risk structure, compare the distances to a marking criteria, mark voxels on a wall of the body lumen in the virtual rendering corresponding to the comparison of the relative distance to the marking criteria, and display the virtual rendering with marking to highlight high risk structures on the display.

11. The system of claim 10 wherein the processor, memory, and display are in an imaging work station.

12. The system of claim 10 further comprising an endoscope operably connected to the processor, whereby the virtual rendering can be registered to a live endoscopic image generated by the endoscope and presented along side of the endoscopic image.

13. A computer program product comprising a non-transitory, computer-readable storage media having encoded thereon:
program instructions for performing a CT scan of the body lumen;
program instructions for segmenting the body lumen;
program instructions for segmenting a high risk structure located outside of the body lumen wall;
program instructions for determining relative distances between the points of the body lumen wall and the high risk structure;
program instructions for comparing the distances to a marking criteria;
program instructions for creating a virtual rendering of the inside of the body lumen corresponding to an interventional camera image; and
program instructions for marking the points of the body lumen wall in the virtual rendering corresponding to the comparison of the relative distance to the marking criteria.

14. The program product of claim 13, wherein the program instructions for marking areas of the body lumen wall comprise instructions for marking areas of the body lumen wall that are within a predefined distance of the high risk structure.

15. The program product of claim 14, wherein the program instructions for marking areas of the body lumen wall comprise instructions for painting voxels on the body lumen wall within a predefined distance to the high risk structure a predefined color.

## Patentansprüche

1. Computerimplementiertes Verfahren zum Hervorheben von Hochrisikostrukturen in einer virtuellen Bildberechnung zur Verwendung während eines Eingriffsverfahrens in ein Körperlumen, umfassend:
Durchführen eines CT-Scans (610) des Körperlumens;
Segmentieren des Körperlumens (630);
Segmentieren einer Hochrisikostruktur, die außerhalb der Körperlumenwand (640) gelegen ist;
Bestimmen von relativen Abständen zwischen den Stellen an der Körperlumenwand und der Hochrisikostruktur (650);
Vergleichen der Abstände mit einem kennzeichnenden Kriterium (655);
Erstellen einer virtuellen Bildberechnung des Inneren des Körperlumens, die einem Kamerabild des Eingriffs entspricht; und
Kennzeichnen der Stellen der Körperlumenwand der virtuellen Bildberechnung des Körperlumens, die dem Vergleich des relativen Abstands zum kennzeichnenden Kriterium (660) entsprechen.

2. Verfahren nach Anspruch 1, wobei Flächen der Körperlumenwand gekennzeichnet werden, die innerhalb eines vordefinierten Abstands der Hochrisikostruktur liegen.

3. Verfahren nach Anspruch 2, wobei Flächen der Körperlumenwand durch Färben von Volumenpixeln innerhalb eines vordefinierten Abstands zur Hochrisikostruktur in einer vordefinierten Farbe gekennzeichnet werden.

4. Verfahren nach Anspruch 3, wobei Flächen der Körperlumenwand in einer von einer Vielzahl von Farben gefärbt werden, die dem Abstand der Hochrisikostruktur zu jeder respektiven Fläche entsprechen, die eine der Vielzahl von vordefinierten Grenzen ist.

5. Verfahren nach Anspruch 3, wobei eine Fläche der Körperlumenwand in einer Farbe gefärbt wird, die einer von einer Vielzahl von Hochrisikostrukturen innerhalb eines vordefinierten Abstands zur Fläche der Körperlumenwand entspricht.

6. Verfahren nach Anspruch 1, wobei jedes Volumenpixel der Körperlumenwand mit einer transparenten Überlagerung gefärbt wird, wenn der Abstand zwischen dem Volumenpixel und einer Hochrisikostruktur innerhalb eines vorbestimmten Schwellwertes liegt.

7. Verfahren nach Anspruch 1, wobei Stellen an der Körperlumenwand gekennzeichnet werden, wenn der Abstand zwischen der Stelle an der Körperlumenwand und der Hochrisikostruktur, gemessen in einem Winkel, in dem ein Arzt geplant hat zu schneiden, innerhalb eines vorbestimmten Schwellwertes liegt.

8. Verfahren nach Anspruch 1, wobei das Körperlumen ein Tracheobronchialbaum ist.

9. Verfahren nach Anspruch 8, wobei die Hochrisikostruktur Blutgefäße sind.

10. System zum Hervorheben von Hochrisikostrukturen in einer virtuellen Bildberechnung zur Verwendung während eines Eingriffsverfahrens in ein Körperlumen, umfassend:
einen Prozessor (310);
einen Speicher (320), der betriebsfähig mit dem Prozessor verbunden ist; und
eine Anzeige (340), die betriebsfähig mit dem Prozessor verbunden ist;
wobei auf dem Speicher ein Modellierprogramm codiert ist, das vom Prozessor ausgeführt wird, um präoperative CT-Scans zu erhalten, aus den präoperativen CT-Scans ein Körperlumen und eine Hochrisikostruktur, die außerhalb der Körperlumenwand liegt, zu segmentieren, eine virtuelle Bildberechnung einer endoskopischen Sicht des Körperlumens zu erzeugen, relative Abstände zwischen den Stellen der Körperlumenwand und der Hochrisikostruktur zu bestimmen, die Abstände zu einem kennzeichnenden Kriterium zu vergleichen, Volumenpixel an einer Wand des Körperlumens in der virtuellen Bildberechnung, die dem Vergleich des relativen Abstands zu dem kennzeichnenden Kriterium entsprechen, zu kennzeichnen und die virtuelle Bildberechnung mit der Kennzeichnung anzuzeigen, um Hochrisikostrukturen auf der Anzeige hervorzuheben.

11. System nach Anspruch 10, wobei der Prozessor, Speicher und die Anzeige eine Arbeitsstation für Bildgebung sind.

12. System nach Anspruch 10, weiter umfassend ein Endoskop, das betriebsfähig mit dem Prozessor verbunden ist, wobei die virtuelle Bildberechnung in einem endoskopischen Livebild, das vom Endoskop erzeugt wird, aufgezeichnet und zusammen mit dem endoskopischen Bild angezeigt werden kann.

13. Computerprogrammprodukt, umfassend ein nichtflüchtiges, computerlesbares Speichermedium, auf dem codiert sind:
Programminstruktionen zum Durchführen eines CT-Scans des Körperlumens;
Programminstruktionen zum Segmentieren des Körperlumens;
Programminstruktionen zum Segmentieren einer Hochrisikostruktur, die außerhalb der Körperlumenwand gelegen ist;
Programminstruktionen zum Bestimmen von relativen Abständen zwischen den Stellen der Körperlumenwand und der Hochrisikostruktur;
Programminstruktionen zum Vergleichen der Abstände mit einem kennzeichnenden Kriterium;
Programminstruktionen zum Erstellen einer virtuelle Bildberechnung des Inneren des Körperlumens, das einem Kamerabild des Eingriffs entspricht; und
Programminstruktionen zum Kennzeichnen der Stellen der Körperlumenwand in der virtuelle Bildberechnung, die dem Vergleich des relativen Abstands zum kennzeichnenden Kriterium entsprechen.

14. Programmprodukt nach Anspruch 13, wobei die Programminstruktionen zum Kennzeichnen von Flächen der Körperlumenwand Instruktionen zum Kennzeichnen von Flächen der Körperlumenwand umfassen, die innerhalb eines vordefinierten Abstands der Hochrisikostruktur liegen.

15. Programmprodukt nach Anspruch 14, wobei die Programminstruktionen zum Kennzeichnen von Flächen der Körperlumenwand Instruktionen zum Färben von Volumenpixeln an der Körperlumenwand innerhalb eines vordefinierten Abstands zu der Hochrisikostruktur in einer vordefinierten Farbe umfassen.

## Revendications

1. Procédé mis en oeuvre par ordinateur pour mettre en évidence des structures à haut risque dans un rendu virtuel pour une utilisation durant une procédure d'intervention dans une lumière corporelle, comprenant :
l'exécution d'une tomodensitométrie (610) de la lumière corporelle ;
la segmentation de la lumière corporelle (630) ;
la segmentation d'une structure à haut risque, qui est située à l'extérieur de la paroi de lumière corporelle (640) ;
la détermination de distances relatives entre les points sur la paroi de lumière corporelle et la structure à haut risque (650) ;
la comparaison des distances à un critère de marquage (655) ;
la création d'un rendu virtuel de l'intérieur de la lumière corporelle correspondant à une image de caméra d'intervention ; et
le marquage des points de la paroi de lumière corporelle du rendu virtuel de la lumière corporelle correspondant à la comparaison de la distance relative au critère de marquage (660).

2. Procédé selon la revendication 1, dans lequel des zones de la paroi de lumière corporelle qui sont à une distance prédéfinie de la structure à haut risque sont marquées.

3. Procédé selon la revendication 2, dans lequel des zones de la paroi de lumière corporelle sont marquées en peignant des voxels dans une couleur prédéfinie à une distance prédéfinie de la structure à haut risque.

4. Procédé selon la revendication 3, dans lequel des zones de la paroi de lumière corporelle sont peintes dans une d'une pluralité de couleurs correspondant à la distance de la structure à haut risque à chaque zone respective étant dans une d'une pluralité de limites prédéfinies.

5. Procédé selon la revendication 3, dans lequel une zone de la paroi de lumière corporelle est peinte dans une couleur correspondant à l'une d'une pluralité de structures à haut risque à une distance prédéfinie de la zone de la paroi de lumière corporelle.

6. Procédé selon la revendication 1, dans lequel chaque voxel de la paroi de lumière corporelle est peint avec un revêtement translucide si la distance entre le voxel et une structure à haut risque est dans une valeur de seuil prédéfinie.

7. Procédé selon la revendication 1, dans lequel des points sur la paroi de lumière corporelle sont marqués si la distance entre le point sur la paroi de lumière corporelle et la structure à haut risque, mesurée à un angle auquel un médecin a l'intention de couper, est dans une valeur de seuil prédéfinie.

8. Procédé selon la revendication 1, dans lequel la lumière corporelle est un arbre respiratoire trachéo-bronchique.

9. Procédé selon la revendication 8, dans lequel la structure à haut risque est des vaisseaux sanguins.

10. Système pour mettre en évidence des structures à haut risque dans un rendu virtuel pour une utilisation durant une procédure d'intervention dans une lumière corporelle, comprenant :
un processeur (310) ;
une mémoire (320) raccordée de manière fonctionnelle au processeur ; et
un écran (340) raccordé de manière fonctionnelle au processeur ;
dans lequel la mémoire a codé sur celle-ci un programme de modélisation, qui est exécuté par le processeur pour recevoir des tomodensitométries avant la procédure, segmenter une lumière corporelle et une structure à haut risque, qui est située à l'extérieur de la paroi de lumière corporelle des tomodensitométries avant la procédure, générer un rendu virtuel d'une vue endoscopique de la lumière corporelle, déterminer des distances relatives entre les points de la paroi de lumière corporelle et la structure à haut risque, comparer les distances à un critère de marquage, marquer des voxels sur une paroi de la lumière corporelle dans le rendu virtuel correspondant à la comparaison de la distance relative au critère de marquage, et afficher le rendu virtuel avec le marquage pour mettre en évidence des structures à haut risque sur l'écran.

11. Système selon la revendication 10, dans lequel le processeur, la mémoire et l'écran sont dans un poste de travail d'imagerie.

12. Système selon la revendication 10, comprenant en outre un endoscope raccordé de manière fonctionnelle au processeur, selon lequel le rendu virtuel peut être enregistré dans une image endoscopique en direct générée par l'endoscope et présenté à côté de l'image endoscopique.

13. Produit de programme informatique comprenant un support de stockage non transitoire lisible par ordinateur ayant codé sur celui-ci :
des instructions de programme pour exécuter une tomodensitométrie de la lumière corporelle ;
des instructions de programme pour segmenter la lumière corporelle ;
des instructions de programme pour segmenter une structure à haut risque située à l'extérieur de la paroi de lumière corporelle ;
des instructions de programme pour déterminer des distances relatives entre les points de la paroi de lumière corporelle et la structure à haut risque ;
des instructions de programme pour comparer les distances à un critère de marquage ;
des instructions de programme pour créer un rendu virtuel de l'intérieur de la lumière corporelle correspondant à une image de caméra d'intervention ; et
des instructions de programme pour marquer les points de la paroi de lumière corporelle dans le rendu virtuel correspondant à la comparaison de la distance relative au critère de marquage.

14. Produit de programme selon la revendication 13, dans lequel les instructions de programme pour marquer des zones de la paroi de lumière corporelle comprennent des instructions pour marquer des zones de la paroi de lumière corporelle qui sont à une distance prédéfinie de la structure à haut risque.

15. Produit de programme selon la revendication 14, dans lequel les instructions de programme pour marquer des zones de la paroi de lumière corporelle comprennent des instructions pour peindre des voxels dans une couleur prédéfinie sur la paroi de lumière corporelle à une distance prédéfinie de la structure à haut risque.
